(19) Europäisches Patentamt
European Patent Office
Office européen des brevets

(11) **EP 3 069 761 A1**

(12) **EUROPEAN PATENT APPLICATION**
published in accordance with Art. 153(4) EPC

(43) Date of publication:
**21.09.2016 Bulletin 2016/38**

(51) Int Cl.:
*A61N 5/06* (2006.01)    *A61B 17/00* (2006.01)
*A61M 25/10* (2006.01)

(21) Application number: **14862673.2**

(22) Date of filing: **14.11.2014**

(86) International application number:
**PCT/JP2014/005744**

(87) International publication number:
**WO 2015/072152 (21.05.2015 Gazette 2015/20)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **14.11.2013 JP 2013235804**

(71) Applicant: **St. Marianna University School of Medicine**
**Kawasaki-shi**
**Kanagawa 216-8511 (JP)**

(72) Inventors:
• **KASHIMURA, Takeshi**
(JP)
• **TAIRA, Yasuhiko**
(JP)
• **NIWAYAMA, Masatsugu**
(JP)

(74) Representative: **Kador & Partner**
**Corneliusstraße 15**
**80469 München (DE)**

(54) **CARBON MONOXIDE POISONING RESOLVING DEVICE, JACKET FOR CARBON MONOXIDE POISONING TREATMENT HAVING SAID DEVICE, AND CATHETER FOR CARBON MONOXIDE POISONING TREATMENT**

(57)     A device for treating carbon monoxide poisoning includes a light emitter that emits light having a wavelength in the range of from 600 to 750 nm. An upper clothing of the present invention emits light having a wavelength in the range of from 600 to 750 nm on the inside of a front body and/or a back body. A catheter of the present invention includes a catheter body that has a distal end portion formed of a light-transmissive material and that includes a plurality of lumens; a light emitter that emits light having a wavelength in the range of from 600 to 750 nm to allow the light to exit through a first lumen from the distal end portion of the catheter body; a balloon that is disposed around a circumferential surface of the distal end portion, that is in communication with a second lumen, and that guides the distal end portion to downstream blood flow in an inflated state; and a pressure sensor that is disposed at the distal end of the distal end portion and that is connected to a cable inserted through a third lumen.

**Fig.8A**

**(Cont. next page)**

Fig.8B

Fig.8

**Description**

TECHNICAL FIELD

The present invention relates to a device for treating carbon monoxide poisoning, the device being used for treating a patient suffering from carbon monoxide poisoning, and an upper clothing and a catheter that include the device for treating carbon monoxide poisoning.

BACKGROUND ART

Carbon monoxide poisoning occurs when carbon monoxide, which is, for example, produced by incomplete combustion occurring such as in fires, is inhaled. Thus, carbon monoxide poisoning is not region specific and can occur in any regions. About over 2,000 of deaths per year are attributed to carbon monoxide poisoning in Japan. It is said that at least tens of thousands of people exhibit no symptoms of the poisoning, but potentially suffer from carbon monoxide poisoning in Japan.

Inhaled carbon monoxide binds to hemoglobin in blood to form carbon monoxide-hemoglobin (CO-Hb). The affinity of carbon monoxide for hemoglobin is known to be about 250-fold higher than the affinity of oxygen for hemoglobin. Thus, inhaled carbon monoxide inhibits the binding of oxygen to hemoglobin to form oxyhemoglobin ($O_2$-Hb). CO-Hb also inhibits the release of oxygen from $O_2$-Hb in peripheral tissues. Thus, inhaled carbon monoxide reduces the oxygen-carrying capacity of hemoglobin and induces tissue hypoxia (see, for example, Non-Patent

Document 1).

As initial therapy for carbon monoxide poisoning, it is effective to immediately remove carbon monoxide from the body by oxygen administration. Methods for administering oxygen to a patient suffering from carbon monoxide poisoning include use of normal breathing to administer oxygen, breathing in concentrated oxygen (for example, 100% oxygen at 1 atmosphere), and hyperbaric oxygen therapy (for example, breathing in 100% oxygen at 2 atmosphere). When the effects of the respective methods for administering oxygen in removing carbon monoxide are compared for half-life of carbon monoxide in blood, the half-life of carbon monoxide is 4 hours in normal breathing, the half-life of carbon monoxide is 40 minutes in breathing in concentrated oxygen, and the half-life of carbon monoxide is 23 minutes in hyperbaric oxygen therapy (see Non-Patent Document 2). This shows that hyperbaric oxygen therapy is very effective in treating carbon monoxide poisoning.

As an alternative to donor blood, a hemoglobin-based artificial oxygen carrier is known (see, for example, Patent Document 1). The artificial oxygen carrier is stored with carbon monoxide bound thereto. The artificial oxygen carrier having carbon monoxide bound thereto is stable in air and thus can be stored for a long period of time.

The carbon monoxide is dissociated by exposure to visible light, and the carbon monoxide is replaced by oxygen by binding oxygen to the oxygen carrier to obtain an artificial oxygen carrier having oxygen bound thereto.

PRIOR ART REFERENCES

PATENT DOCUMENTS

[Patent Document 1] Japanese Unexamined Patent Application Publication No. 2007-045718

NON-PATENT DOCUMENTS

[Non-Patent Document1] E. Narimatsu, Y. Asai, "2. Carbon Monoxide Poisoning", Clinic All-Round an extra issue, 2002. 5, Vol. 51, p. 748-751
[Non-Patent Document2] K. Iseki, C. Tase, "Treatment of Acute Poisoning", Japanese Journal of Intensive Care Medicine, 2002, 26(5), p.329-333

SUMMARY OF THE INVENTION

PROBLEMS TO BE SOLVED BY THE INVENTION

Hyperbaric oxygen therapy requires administration of highly concentrated oxygen (100% oxygen) under a high atmosphere environment (at 2 atmosphere), and thus the therapy is done by using a large hyperbaric oxygen therapy chamber. However, the hyperbaric oxygen therapy chamber is expensive and requires operation by a doctor, a nurse, and a technician, and thus only about 50 medical facilities have the chamber in Japan. Thus, it is very difficult to promptly provide appropriate initial therapy to patients suffering from carbon monoxide poisoning throughout the country.

The present invention has been developed in view of the foregoing and has an object to provide a device for treating carbon monoxide poisoning, the device allowing medical facilities in various regions to use the cheaper device to provide effective initial therapy for patients suffering from carbon monoxide poisoning, and an upper clothing and a catheter that include the device for treating carbon monoxide poisoning.

MEANS FOR SOLVING THE PROBLEMS

As described above, carbon monoxide binding to a hemoglobin-based artificial oxygen carrier is dissociated by exposure to visible light. This suggests that light exposure may also result in dissociation of carbon monoxide from CO-Hb in patients suffering from carbon monoxide poisoning. Thus, the inventors of the present invention have found that exposure of CO-Hb of rats to light resulted in dissociation of carbon monoxide from the CO-Hb. And it is expected that exposure of blood of patients suffering from carbon monoxide poisoning to light can result in dissociation of carbon monoxide from the CO-Hb. However, hemoglobin has high affinity for carbon monoxide as described above, and thus even if carbon monoxide is dissociated in blood, the carbon monoxide may bind to the hemoglobin again before oxygen binds to the hemoglobin.

As a result of further research based on the above finding, the inventors of the present invention have also found that it is effective to directly or indirectly expose blood to light immediately before the blood flows into lung, thereby achieving the present invention.

Thus, the present invention relates to the following device for treating carbon monoxide poisoning:

[1] A device for treating carbon monoxide poisoning, the device including a light emitter that emits light having a wavelength in the range of from 600 to 750 nm.

The present invention also relates to the following upper clothing:

[2] An upper clothing for subjecting a patient suffering from carbon monoxide poisoning to light radiation to treat the carbon monoxide poisoning, the upper clothing including the device for treating carbon monoxide poisoning according to [1], wherein the light emitter emits light having a wavelength in the range of from 600 to 750 nm on the inside of a front body and/or on the inside of a back body.

[3] The upper clothing according to [2], wherein the light emitter includes a plurality of light guides that allow light emitted by a light source disposed outside of the upper clothing to enter from one end and allow the entering light to exit from light transmitting portions at the other end, and wherein the plurality of light transmitting portions are disposed on the inside of the front body and/or on the inside of the back body.

The present invention further related to the following catheter:

[4] A catheter for insertion into a blood vessel of a patient suffering from carbon monoxide poisoning and for transmission of light into the blood, the catheter including the device for treating carbon monoxide poisoning according to [1], wherein the light emitter emits light having a wavelength in the range of from 600 to 750 nm at the distal end of the catheter.

[5] The catheter according to [4], wherein the catheter includes a catheter body that has a distal end portion formed of a light-transmissive material and that includes a first lumen, a second lumen, and a third lumen; the light emitter that emits light having a wavelength in the range of from 600 to 750 nm to allow the light to exit through the first lumen from the distal end portion of the catheter body; a balloon that is disposed around a circumferential surface of the distal end portion, that is in communication with the second lumen, and that guides the distal end portion to downstream blood flow in an inflated state; and a pressure sensor that is disposed at the distal end of the distal end portion, that is connected to a cable inserted through the third lumen, and that measures intracardiac or intravascular pressure.

[6] The catheter according to [5], wherein the distal end portion is a portion from the distal end of the catheter body to a position at 10 to 15 cm from the distal end.

[7] The catheter according to [5] or [6], wherein the light emitted by the light emitter is transmitted outside of the catheter body in a distal-end side, a central portion, and a proximal-end side of the distal end portion, excluding the portion with the balloon disposed thereon.

[8] The catheter according to any one of [5] to [7], wherein the light emitter includes a plurality of light guides that allow light emitted by a light source disposed outside of the catheter body to enter from one end and allow the entering light to exit from light transmitting portions at the other end, and wherein the plurality of light transmitting portions are disposed in the distal-end side, the central portion, and the proximal-end side of the distal end portion.

[9] The catheter according to any one of [5] to [7], wherein the light emitter includes a plurality of LEDs, and wherein the plurality of LEDs are disposed in the distal-end side, the central portion, and the proximal-end side of the distal end portion.

[10] The catheter according to any one of [4] to [9], wherein the light emitted by the light emitter has a light intensity of 1 mW or more.

EFFECTS OF THE INVENTION

The present invention allows provision of effective and inexpensive initial therapy for patients suffering from carbon monoxide poisoning in any regions.

BRIEF DESCRIPTION OF THE DRAWINGS

FIG. 1 is a schematic view of a catheter according to an Embodiment 1.
FIGs. 2A-C are a view illustrating a structure of a distal end portion of a catheter body of a catheter according to the Embodiment 1.
FIGs. 3A-C are a graph illustrating the relationship between wavelength of light and energy absorbed by carbon monoxide-hemoglobin.
FIGs. 4A-C are a cross-sectional view of a distal end portion of a catheter body according to a modification of the Embodiment 1.
FIGs. 5A-C are a cross-sectional view of a distal end portion of a catheter body according to another modification.
FIGs. 6A-C are a view illustrating a structure of a distal end portion of a catheter according to an Embodiment 2.

FIGs. 7A-C are a view illustrating a structure of a distal end portion of a catheter according to a modification of the Embodiment 2.

FIGs. 8A and 8B are a view illustrating a structure of an upper clothing according to an Embodiment 3.

FIGs. 9A and 9B are a view illustrating a structure of an upper clothing according to a modification of the Embodiment 3.

FIG. 10 is a graph illustrating the relationship between light exposure time and saturation of carbon monoxide-hemoglobin.

FIG. 11 is a graph illustrating the relationship between light exposure time and saturation of carbon monoxide-hemoglobin.

FIG. 12 is a graph illustrating the relationship between light exposure time and saturation of carbon monoxide-hemoglobin.

FIG. 13 is a graph illustrating the relationship between light exposure time and saturation of carbon monoxide-hemoglobin.

FIG. 14 is a graph illustrating the relationship between light exposure time and saturation of carbon monoxide-hemoglobin.

MODE FOR CARRYING OUT THE INVENTION

Now, a device for treating carbon monoxide poisoning according to the present invention will be described with reference to the accompanying drawings. The device for treating carbon monoxide poisoning includes a light emitter that emits light having a wavelength in the range of from 600 to 750 nm. The device for treating carbon monoxide poisoning according to the present invention is mainly used for treatment of a patient suffering from acute carbon monoxide poisoning and exposes blood flowing through the pulmonary artery to a light having a predetermined wavelength. In the Embodiments 1 and 2, a catheter that includes the device for treating carbon monoxide poisoning will be described, while in the Embodiment 3, an upper clothing that includes the device for treating carbon monoxide poisoning will be described.

[Embodiment 1]

(Structure of Catheter)

Now, a catheter according to the present invention will be described in detail with reference to the accompanying drawings. The catheter according to the present invention is for insertion into a blood vessel of a patient suffering from carbon monoxide poisoning and for exposure of the blood to light and can be inserted in a manner similar to pulmonary artery catheters. For example, the distal end of the catheter according to the present invention is inserted through the right internal jugular vein and is advanced to the superior vena cava, the right atrium, the right ventricle, and the pulmonary artery. Then, the distal end of the catheter is deployed adjacent to the pulmonary artery and exposes the blood flowing through the pulmonary artery to light having a predetermined wavelength.

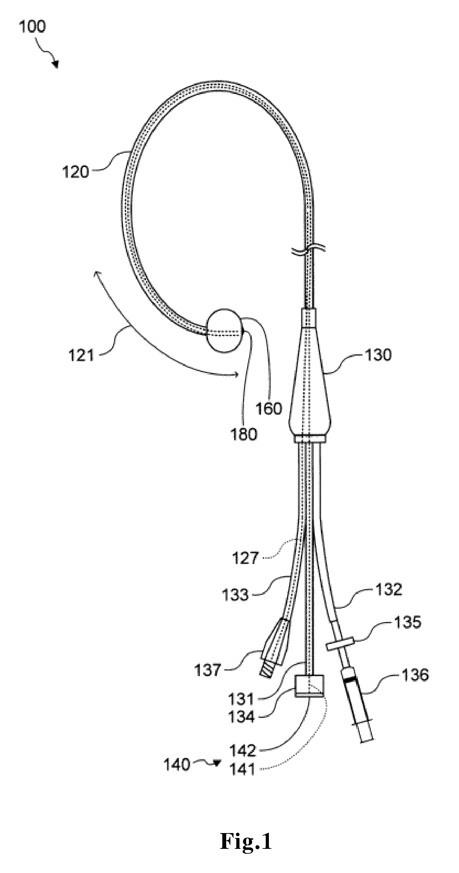

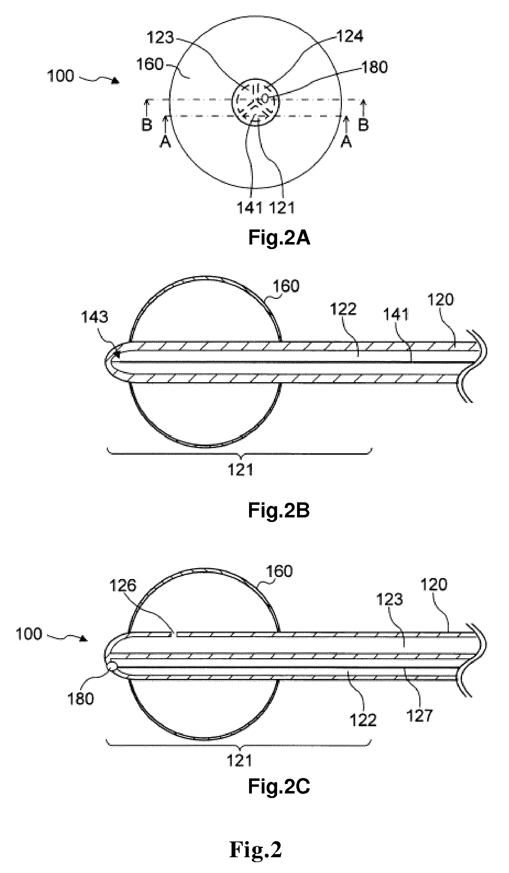

FIGs. 1 and 2 are a view illustrating the structure of a catheter 100 according to the Embodiment 1 of the present invention. FIG. 1 is a schematic view of the catheter 100 according to the Embodiment 1. FIG. 2A is a view of the catheter 100, as seen from the distal end. FIG. 2B is a cross-sectional view taken along the line A-A in FIG. 2A. FIG. 2C is a cross-sectional view taken along the line B-B in FIG. 2A.

As illustrated in FIGs. 1 and 2, the catheter 100 includes a catheter body 120, a light emitter 140, a balloon 160, and a pressure sensor 180.

The catheter body 120 is an elongated tube that is partially inserted into a blood vessel to connect the inside of the blood vessel to the outside of the blood vessel. The catheter body 120 includes a distal end portion 121, a first lumen 122, a second lumen 123, and a third lumen 124. The catheter body 120 is curved with a predetermined radius of curvature (see FIG. 1). Preferably, the catheter body 120 has a softness that allows the body to bend enough to pass through the right internal jugular vein to the pulmonary artery. This allows the distal end of the catheter body 120 to be smoothly advanced through the superior vena cava, the right atrium, and the right ventricle to the pulmonary artery. The circumferential surface of catheter body 120 may be coated with, for example, heparin. This can prevent the formation of blood clots on the circumferential surface of the catheter body 120 in the blood vessel. The outer diameter of the catheter body 120 is not restricted as long as the distal end of the catheter 100 can be deployed in the pulmonary artery. The catheter body 120 has an outer diameter of from about 5Fr to 8Fr (about 1.6 mm to 2.6 mm).

The distal end portion 121 is a distal end region of the catheter body 120. The distal end portion 121 includes a light transmitting portion 143 of the light emitter 140 that emits light having a predetermined wavelength. The distal end portion 121 is a portion from the distal end of the catheter body 120 to a position at 10 to 15 cm from the distal end.

The material of the distal end portion 121 is not restricted as long as the material can transmit light having a predetermined wavelength. The material of the distal end portion 121 is, for example, polyvinyl chloride or polyurethane resin. In the embodiment, the entire portions of the catheter body 120, including the distal end portion 121, are formed of light-transmissive polyvinyl chloride or polyurethane resin. Thus, in the embodiment, the entire portions of the catheter body 120, including the distal end portion 121, preferably have a softness (flexibility) that allows the portions to bend enough to pass through the right internal jugular vein to the pulmonary artery.

The size and the shape of the radial cross-section of the first lumen 122, the second lumen 123, and the third lumen 124 are not restricted. In the embodiment, the radial cross-section of the first lumen 122, the second lumen 123, and the third lumen 124 has a size that is about one third of the size of the radial cross-section of the catheter body 120. In the embodiment, the radial cross-section of the first lumen 122, the second lumen 123, and the third lumen 124 has a shape of a sector that is one third of a circle.

The first lumen 122 includes a light guide 141. The distal end of the first lumen 122 in the catheter body 120 is closed. This prevents the blood from flowing into the first lumen 122.

The second lumen 123 is a pathway for gas supplied to the balloon 160. The second lumen 123 is in communication with the balloon 160 via a through-hole 126 disposed in the inner wall of the second lumen 123. The distal end of the second lumen 123 in the catheter body 120 is also closed. This prevents the blood from flowing into the second lumen 123.

The third lumen 124 includes a cable 127 connected to the pressure sensor 180. In the distal end of the third lumen 124, the pressure sensor 180 is incorporated. The distal end of the third lumen 124 in the catheter body 120 is also closed. This prevents the blood from flowing into the third lumen 124.

To the proximal end of the catheter body 120, a light guide lumen 131, a balloon lumen 132, and a pressure sensor lumen 133 are connected via a connector 130. The light guide lumen 131, the balloon lumen 132, and the pressure sensor lumen 133 are a hollow tube.

The light guide lumen 131 includes the light guide 141. One end of the light guide lumen 131 is connected to the first lumen 122 via the connector 130, and the other end is connected to a light source connector 134. The light source connector 134 optically connects the light guide 141 to a light source 142.

The balloon lumen 132 is a pathway for gas supplied to the balloon 160. One end of the balloon lumen 132 is connected to the second lumen 123 via the connector 130, and the other end is connected to a balloon inflation valve 135. The balloon inflation valve 135 can be connected only to a syringe 136 having a volume that corresponds to the volume of gas supplied to the balloon 160.

The pressure sensor lumen 133 includes the cable 127 connected to the pressure sensor 180. One end of the pressure sensor lumen 133 is connected to the third lumen 124 via the connector 130, and the other end is connected to a connector 137 connected to a monitor (not shown). The connector 137 electrically connects the cable 127 to the monitor.

The light emitter 140 transmits light through the inside to the outside of the catheter body 120 in the distal end portion 121 (at the distal end) of the catheter body 120. As described below, the catheter 100 is used by deploying the distal end of the catheter body 120 in blood. If heat was generated in the portion deployed in a blood vessel, the blood components might be modified. Thus, the portion of the catheter 100 to be deployed in a blood vessel preferably generates no heat. The structure of the light emitter 140 is not restricted as long as the portion of the catheter 100 to be deployed in a blood vessel generates no heat. In the embodiment, the light emitter 140 includes the light guide 141 and the light source 142.

The light guide 141 allows light emitted by the light source 142 to enter from one end and allows the light to exit from the distal end in the distal end portion 121. The light guide 141 extends in the first lumen 122, the connector 130, and the light guide lumen 131 of the catheter body 120. The light guide 141 serves the above functions and preferably has a softness that allows the guide to bend enough to pass through the right internal jugular vein to the pulmonary artery during use. Examples of the light guide 141 include optical fibers and silica fibers. In the embodiment, the light guide 141 is an optical fiber, and the light transmitting portion 143 of the light guide 141 is disposed at the distal end of the catheter body 120 (see FIG. 2B).

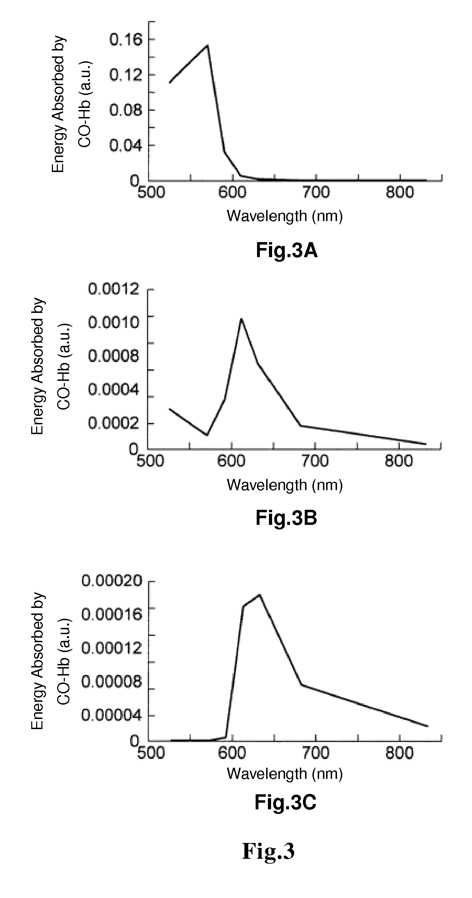

The inventors of the present invention conducted studies to find a wavelength of light that should be transmitted through the light transmitting portion 143 (distal end) of the light guide 141. Transmission of light in biological tissues (medium) containing CO-Hb, $O_2$-Hb, Hb, and water as absorbers was analyzed by a Monte Carlo method. For scattering coefficients, the inventors referred to Steven L Jacques, "Optical properties of biological tissues: a review", Phys. Med. Biol. 58, 2013, pp. R37-R61. For absorption coefficient, the inventors referred to W. G. Zijlstra, A. Buursma, O. W. Van Assendelft, Visible and near infrared absorption spectra of human and animal Haemoglobin determination and application, 2000, pp. 58-59 and W. G. Zijistra, A. Buursms, W. P. Meeuwsen, "Absorption Spectra of Human Fetal and Adult Oxyhemoglobin, De-Oxyhemoglobin, Carboxyhemoglobin, and Methemoglobin", CLINICAL CHEMISTRY, Vol. 37, 9, 1991, pp. 1633-1638. The results of the analysis by a Monte Carlo method are shown in FIG. 3.

FIG. 3A is a graph illustrating the relationship between the wavelength of transmitted light and the light energy absorbed by CO-Hb in a blood vessel without transmission through a tissue. FIG. 3B is a graph illustrating the relationship between the wavelength of transmitted light and the light energy absorbed by CO-Hb in a blood vessel after transmission through a 5 mm tissue. FIG. 3C is a graph illustrating the relationship between the wavelength of transmitted light and the light energy absorbed by CO-Hb in a blood vessel after transmission through a 10 mm tissue. In FIGs. 3A-C, the wavelength (nm) of transmitted light is taken along the abscissa, and the energy (a.u.) of light absorbed by CO-Hb in a blood vessel is taken along the ordinate. In the graphs, the values of the ordinate vary significantly because the values depend on the thickness of the tissue through which light was transmitted.

As illustrates in FIGs. 3A to 3C, the wavelength of light that provides a penetration depth (a depth at which the light intensity is 1/e) of from 1 to 2 mm or more has been found to be 600 nm or more. It has been also found that light having a wavelength of about from 600 to 1000 nm can be transmitted through more blood in a blood vessel or a capillary bed. It is also expected that in the case of transmission of strong light having a short wavelength, absorption of the light energy is concentrated in blood in the regions about 1 mm away from a transmitting surface, and thus thermal damage to blood cells may be caused.

To allow CO-Hb to absorb more light energy, the light should have somewhat high susceptibility to absorption by CO-Hb. The wavelength of light having somewhat high susceptibility to absorption by CO-Hb was derived from earlier studies reported in literature. The studies indicate that light having a wavelength of 750 nm or more is less susceptible to absorption by CO-Hb and that sufficient energy is not transferred. These indicate that the appropriate wavelength of light that allows light energy to be transferred widely and to be efficiently absorbed by CO-Hb is in the range of from 600 to 750 nm. By way of example, light having a wavelength of 680 nm was used in experiments described below, because the light has a penetration depth of from 1 to 2 mm and an absorption coefficient of about 0.01/mm and is expected to act on blood located relatively deep. As described above, light having a wavelength in the range of from 600 to 750 nm can effectively dissociate carbon monoxide from CO-Hb (carbon monoxide-hemoglobin). Thus, the light transmitted through the light transmitting portion 143 (distal end) of the light guide 141 preferably has a wavelength in the range of from 600 to 750 nm.

The type of the light source 142 is not restricted. Examples of the light source 142 include LEDs and cold lamps. The light emitted by the light source 142 enters from a surface of the proximal end of the light guide 141, then the light is guided within the light guide 141, and the light exits from the light transmitting portion 143. The light emitted by the light source 142 may have any illuminance at blood to be exposed as long as the light can dissociate carbon monoxide from CO-Hb. In the embodiment, the blood is preferably exposed to light at an illuminance of 100,000 lux or more. If the blood was exposed to light at an illuminance of less than 100,000 lux, carbon monoxide might not be dissociated from CO-Hb. The light transmitted through the light transmitting portion 143 preferably has an intensity that does not affect the living body. In particular, the light transmitted through the light transmitting portion 143 preferably has an intensity of 1 mW or more. If the light transmitted through the light transmitting portion 143 had an intensity of less than 1 mW, carbon monoxide might not be dissociated from CO-Hb.

The balloon 160 guides the distal end of the catheter body 120 to downstream blood flow. The balloon 160 is disposed around part of a circumferential surface of the distal end portion of the catheter body 120. The volume of the balloon 160 is not restricted and usually about 0.7 mL to 1.5 mL. The material of the balloon 160 is not restricted and usually natural rubber or the like. When the plunger of the syringe 136 is pushed, the gas in the syringe 136 is allowed to flow through the balloon lumen 132, the connector 130, and the second lumen 123 into the balloon 160 to inflate the balloon 160.

The pressure sensor 180 is disposed at the distal end of the distal end portion 121 and detects intracardiac or intravascular pressure to provide an indication of the location of the distal end of the catheter 100. The pressure sensor 180 is connected to the cable 127.

The inventors of the present invention have found that exposure of CO-Hb to light having a predetermined wavelength can result in effective dissociation of carbon monoxide from CO-Hb. In the living body, the blood flows through the right atrium into the heart and flows through the right ventricle into the lung. The lung exchanges carbon dioxide in the blood from the heart and inhaled oxygen. Thus, the inventors of the present invention assumed that dissociation of carbon monoxide from CO-Hb immediately before the blood enters the lung could result in efficient removal of carbon monoxide from the body of a patient suffering from acute carbon monoxide poisoning using the functions of the lung. However, strong light delivered from outside the body cannot be efficiently transmitted to the blood before the blood flows into the lung. Thus, it is necessary that the light transmitting portion 143 that transmits light be disposed within the body. Use of the catheter 100 that allows the light transmitting portion 143 to be inserted into the pulmonary artery just proximal to the lung provides effective removal of carbon monoxide.

(How to Use Catheter)

The catheter 100 according to the present invention can be used, for example, in the following manner. The catheter 100 of the present invention is a pulmonary artery catheter, and thus is inserted from, for example, the right internal jugular vein. First, local anesthesia is provided to a site for insertion of the catheter 100. Next, a guide wire is inserted into the blood vessel, and then the catheter 100 is inserted over the guide wire. After the catheter 100 is inserted a predetermined distance, the balloon 160 is inflated. The catheter 100 is inserted while monitoring the intravascular or intracardiac pressure using the pressure sensor 180 disposed at the distal end of the catheter body 120. The balloon 160 travels through the blood stream, which allows the distal end of the catheter 100 to advance through the right atrium, the right ventricle, and the pulmonary artery. Preferably, the distal end of the catheter 100 is deployed adjacent to the alveoli. The light having a wavelength in the range of from 600 to 750 nm is transmitted through the light transmitting portion 143, leaving the distal end of the catheter 100 indwelling in a predetermined location. The light transmitting time is not restricted. The light transmitting time is adjusted depending on the symptoms of the patient and the concentration of carbon monoxide in blood.

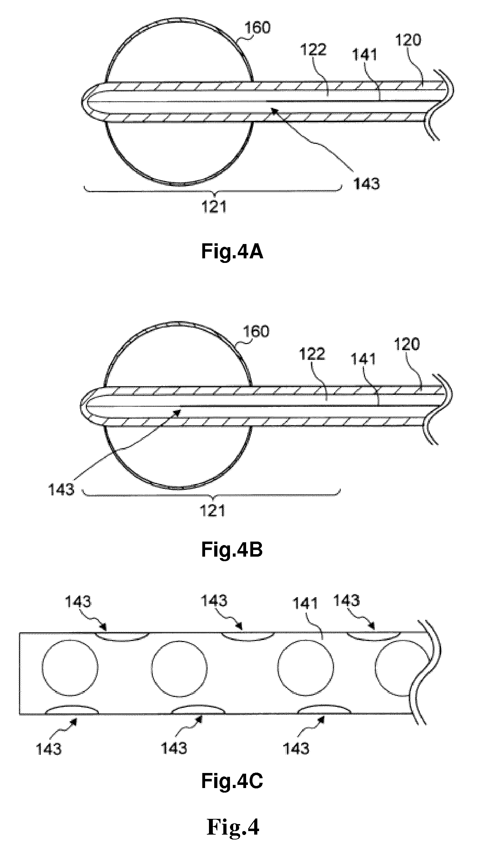

Although the light transmitting portion 143 is disposed at a distal-end side of the catheter body 120 in the embodiment, the light transmitting portion 143 may be disposed in a proximal-end side of the catheter body 120, the side being proximal from the balloon 160 (FIG. 4A) and may be disposed within the balloon 160 (FIG. 4B). If the light transmitting portion 143 is a portion from the distal end of the catheter body 120 to a position at 10 to 15 cm from the distal end, excluding the portion with the balloon 160 disposed thereon, light emitted by the light emitter 140 is not blocked by the balloon 160, which can efficiently expose CO-Hb to the light and thus can efficiently dissociate carbon monoxide from CO-Hb. The light guide 141 may includes a plurality of light transmitting portions 143 (FIG. 4C). In this case, the plurality of light transmitting portions 143 are disposed around a circumferential surface of the light guide 141. In the embodiment, the plurality of light transmitting portions 143 are formed by partially removing the coating of the optical fiber.

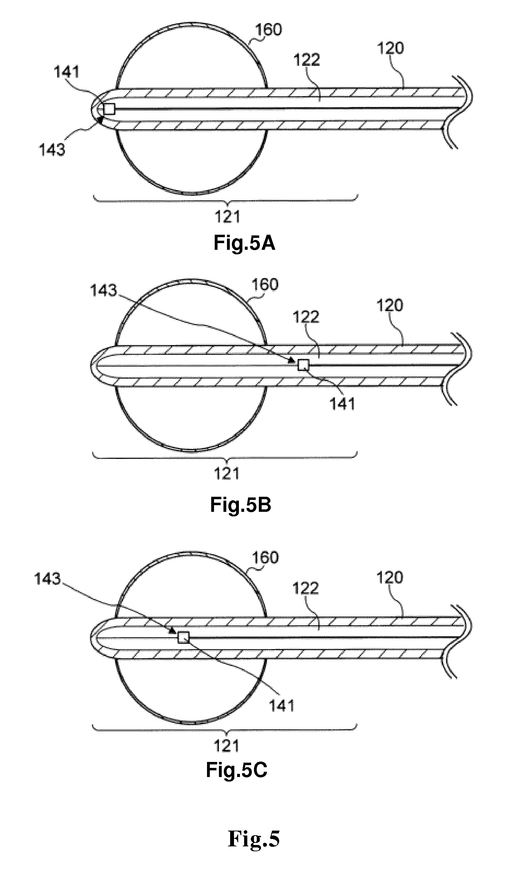

Although, in the embodiment, use of an optical fiber and the light source 142 as the light emitter 140 is described by way of example, an LED may be disposed at the distal end of the catheter body 120 as the light emitter 140, as illustrated in FIG. 5A. In this case, the light emitter 140 includes an LED and a power source. The light emitting surface (the light transmitting portion 143) of the LED (the light guide 141) is disposed at the distal end of the catheter body 120. The LED is electrically connected to the power source (not shown). Also in this case, the LED may be disposed in a proximal-end side of the catheter body 120, the side being proximal from the balloon 160 (FIG. 5B) and may be disposed within the balloon 160 (FIG. 5C).

[Embodiment 2]

A catheter 200 according to the Embodiment 2 differs from the catheter 100 according to the Embodiment 1 in, for example, the structure of a catheter body 220. Similar reference numerals are used to denote components similar to the components of the catheter 100 according to the Embodiment 1, and the components are not described here.

(Structure of Catheter)

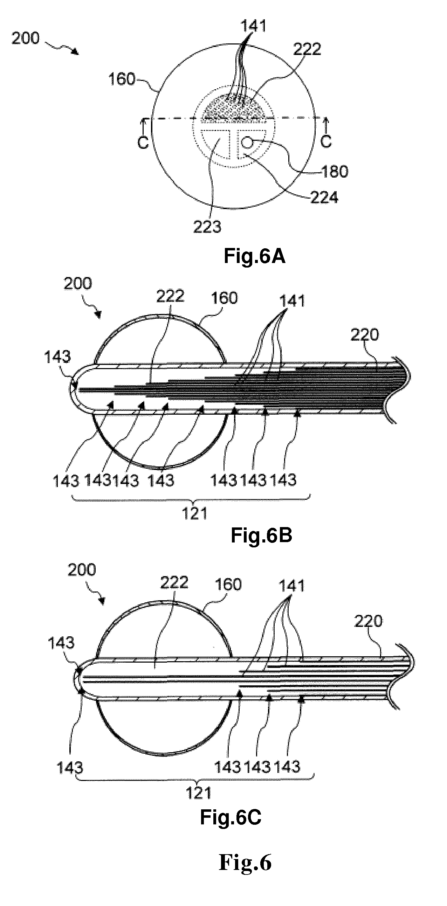

FIG. 6 is a view illustrating a structure of the catheter 200 according to the Embodiment 2. FIG. 6A is a view of the catheter body 220, as seen from the distal end. FIG. 6B is a cross-sectional view taken along the line C-C in FIG. 6A. FIG. 6C illustrates another example of the arrangement of light guides 141.

As illustrated in FIG. 6A, the catheter body 220 of the catheter 200 according to the Embodiment 2 includes a first lumen 222, a second lumen 223, and a third lumen 224. In the embodiment, the radial cross-section of the first lumen 222 has a size that is about half of the size of the lumen of the catheter body 220. The radial cross-section of the second lumen 223 and the third lumen 224 has a size that is one fourth of the lumen of the catheter body 220.

The first lumen 222 includes the plurality of light guides 141. The proximal end of the plurality of light guides 141 is optically connected to a light source 142. As illustrated in FIG. 6B, the plurality of light guides 141 are disposed so that light transmitting portions 143 (end surfaces) are positioned across a distal end portion 121. Then, light through the light transmitting portions 143 are transmitted outside of the catheter body through a distal-end side, a central portion, and a proximal-end side of the distal end portion 121. As illustrated in FIG. 6C, the light guides 141 may be disposed so that light through the light transmitting portions 143 is transmitted outside of the catheter body through a distal-end side, a central portion, and a proximal-end side of the distal end portion 121, excluding the portion with the a balloon disposed thereon.

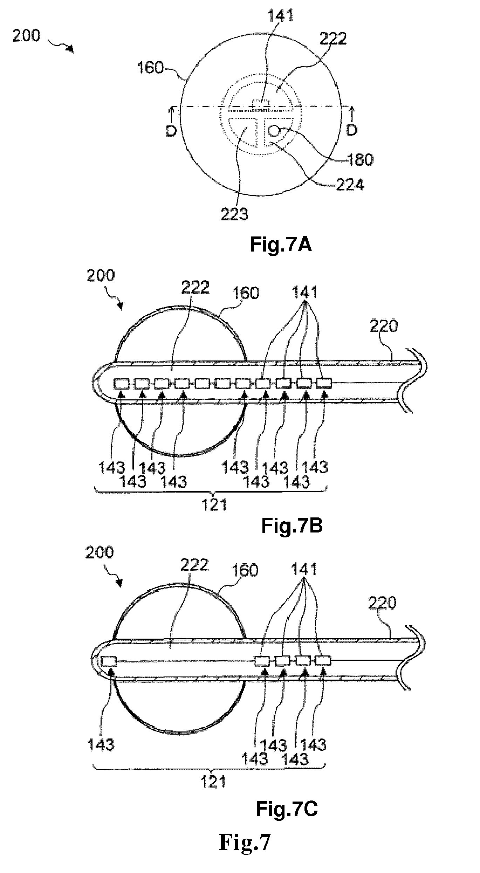

FIGs. 7A-C are a view illustrating a structure of a distal end portion of a catheter 200 according to a modification of the Embodiment 2. FIG. 7A is a view of a catheter body 220, as seen from the distal end. FIG. 7B is a cross-sectional view taken along the line D-D in FIG. 7A. FIG. 7C illustrates another example of the arrangement of light guides 141.

As illustrated in FIGs. 7, a plurality of LEDs may be used in place of a plurality of optical fibers as the plurality of light guides 141. In this case, the plurality of LEDs (light guides 141) are disposed so that light emitting portions 143 (emitting surfaces) are positioned across a distal end portion 121. Then, light from the light emitting portions 143 is transmitted outside of the catheter body through a distal-end side, a central portion, and a proximal-end side of the distal end portion 121. As illustrated in FIG. 7C, the LEDs may be disposed so that light from the light emitting portions 143 is transmitted outside of the catheter body 220 through the distal-end side, the central portion, and the proximal-end side of the distal end portion 121, excluding the portion with the a balloon 160 disposed thereon. In these cases, the LEDs in the distal end portion 121 are arranged in series and are electrically connected to a power source (not shown). In this manner, transmission of light through the distal end portion 121 excluding the portion with the balloon 160 disposed thereon allows efficient exposure of the blood to light having a predetermined wavelength and efficient dissociation of carbon monoxide from CO-Hb. The above arrangement can also reduce the number of the light guides 141.

[Embodiment 3]

(Structure of Upper Clothing)

Now, an upper clothing according to the present invention will be described with reference to the accompanying drawings. The upper clothing according to the present invention is an upper clothing for subjecting a patient suffering from carbon monoxide poisoning to light radiation to treat the carbon monoxide poisoning and can be worn in a similar manner to clothings such as vests, shirts, and sweaters. Thus, the upper clothing according to the present invention transmits light having a predetermined wavelength to the entire pulmonary vascular bed, which allows for gas exchange with air outside the body, when the patient wears the clothing. Preferably, the upper clothing is put directly on the body in order to transmit light to the blood in the blood vessel.

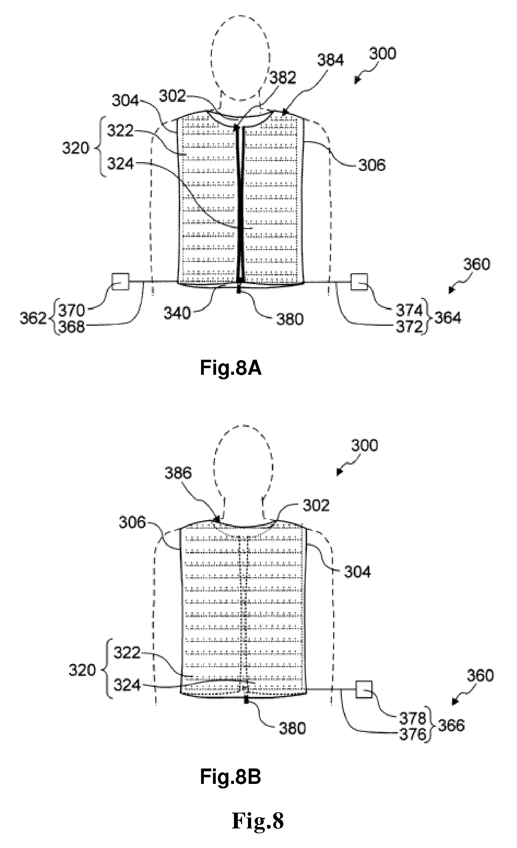

FIGs. 8 are a view illustrating a structure of an upper clothing 300 according to the Embodiment 3 of the present invention. FIG. 8A and FIG. 8B are a front view and a back view of the upper clothing 300, respectively.

As illustrated in FIGs. 8, the upper clothing 300 includes a front body 320, a back body 340, and light emitters 360 that include a first light emitter 362, a second light emitter 364, and a third light emitter 366. The configuration of the upper clothing 300 is not restricted. Examples of the configuration of the upper clothing include front opening configurations, front closing configurations, sleeved configurations, sleeveless configurations, and combinations thereof. In the embodiment, the upper clothing 300 is a sleeveless clothing with a front opening. In particular, the front body 320 and the back body 340 are connected to each other at a right shoulder portion, a left shoulder portion, a right underarm portion, and a left underarm portion when the clothing is worn. To put on the upper clothing 300, the head is inserted through a first opening 302 disposed between the right shoulder portion and the left shoulder portion, the right arm is inserted through a second opening 304 disposed between the right shoulder portion and the right underarm portion, and the left arm is inserted through a third opening 306 disposed between the left shoulder portion and the left underarm portion. The material of the clothing is also not restricted. Examples of the material include cotton, hemp, polyester, acrylic resin, polyurethane, and rayon.

The front body 320 is positioned on the stomach side (front side), as seen by a wearer, when the clothing is worn. The front body 320 includes the first light emitter 362 and the second light emitter 364. The configuration of the front body 320 is not restricted. The front body 320 may or may not include left and right bodies. In the embodiment, the front body 320 includes left and right bodies, which are a first front body 322 and a second front body 324. The first front body 322 and the second front body 324 are configured to be connected to each other by a zipper 380. This makes the upper clothing 300 easy to put on and take off. The first front body 322 is positioned on the left side when the clothing is worn. The first front body 322 includes the first light emitter 362. The second front body 324 is positioned on the right side when the clothing is worn. The second front body 324 includes the second light emitter 364.

The back body 340 is positioned on the back side, as seen by a wearer, when the clothing is worn. The back body 340 includes the third light emitter 366.

The light emitters 360 emit light toward the entire chest on the inside of the upper clothing 300. The light emitters 360 include the first light emitter 362, the second light emitter 364, and the third light emitter 366. As described below, the upper clothing 300 is worn by a patient during use. If the light transmitting portions generated heat, the wearer would be burned, and thus the portions that face the wearer preferably generate no heat. The configuration of the first light emitter 362, the second light emitter 364, and the third light emitter 366 is not restricted as long as the portions that face the wearer generate no heat. In the embodiment, the first light emitter 362 includes a plurality of first light guides 368 and a first light source 370. The second light emitter 364 includes a plurality of second light guides 372 and a second light source 374. The third light emitter 366 includes a plurality of third light guides 376 and a third light source 378.

The first light guides 368, the second light guide 372, and the third light guides 376 allow light respectively emitted by the first light source 370, the second light source 374, and the third light source 378 disposed outside of the front body 320 and the back body 340 to enter from one end and allow the light to exit from the distal end on the inside of the upper clothing 300. The first light guides 368, the second light guides 372, and the third light guides 376 are disposed on the inside of the upper clothing 300. Preferably, the first light guides 368, the second light guides 372, and the third light guides 376 serve the above functions and preferably have an appropriate softness. Examples of the first light guides 368, the second light guides 372, and the third light guides 376 include optical fibers and silica fibers. In the embodiment, the first light guides 368, the second light guides 372, and the third light guides 376 are an optical fiber. There may be a single first light guide 368, a single second light guide 372, and a single third light guides 376 as long as the guides achieve a desired illuminance of transmitted light as described below. The first light guides 368 are disposed on the inside of the first front body 322, the second light guides 372 are disposed on the inside of the second front body 324, and the third light guides 376 are disposed on the inside of the back body 340. The first light guides 368, the second light guides 372, and the third light guides 376 may be configured to transmit light through the distal end only in an upper portion of the inside of the upper clothing 300 (a portion adjacent to a location that corresponds to a location of the wearer's pulmonary artery).

The type of the first light source 370, the second light source 374, and the third light source 378 is not restricted. Examples of the first light source 370, the second light source 374, and the third light source 378 include LEDs and cold lamps. Light emitted by the first light source 370 enters from a surface of the proximal end of the first light guides 368, then the light is guided within the light guides 368, and the light exits from first light transmitting portions 382. Light emitted by the second light source 374 enters from a surface of the proximal end of the second light guides 372, then the light is guided within the light guides 372, and the light exits from second light transmitting portions 384. Light emitted by the third light source 378 enters from a surface of the proximal end of the third light guides 376, then the light is guided within the light guides 376, and the light exits from third light transmitting portions 386. There may be a single light source. In this case, the single light source is optically connected to a plurality of first light guides 368, a plurality of second light guides 372, and a plurality of third light guides 376.

The illuminance of light emitted by the first light source 370, the second light source 374, and the third light source 378 is not restricted as long as the light can dissociate carbon monoxide from CO-Hb. In the embodiment, the blood is preferably exposed to light emitted by the first light source 370, the second light source 374, and the third light source 378 at an illuminance of 100,000 lux or more. Exposure to light at an illuminance of less than 100,000 lux may not result in dissociation of carbon monoxide from CO-Hb. In the case, the blood is exposed to light at an illuminance of about 500,000 lux.

(How to Use Upper Clothing)

The upper clothing 300 according to the present invention may be used in, for example, the following manner. The upper clothing 300 of the present invention is put on a wearer in a manner similar to common clothings so that the first light transmitting portions 382, the second light transmitting portions 384, and the third light transmitting portions 386 are positioned in a predetermined location. In the state, the upper clothing 300 transmits light having a wavelength in the range of from 600 to 750 nm through the first light transmitting portions 382, the second light transmitting portions 384, and the third light transmitting portions 386. The light transmitting time is not restricted. The light transmitting time is adjusted depending on the symptoms of the patient and the concentration of carbon monoxide in the blood.

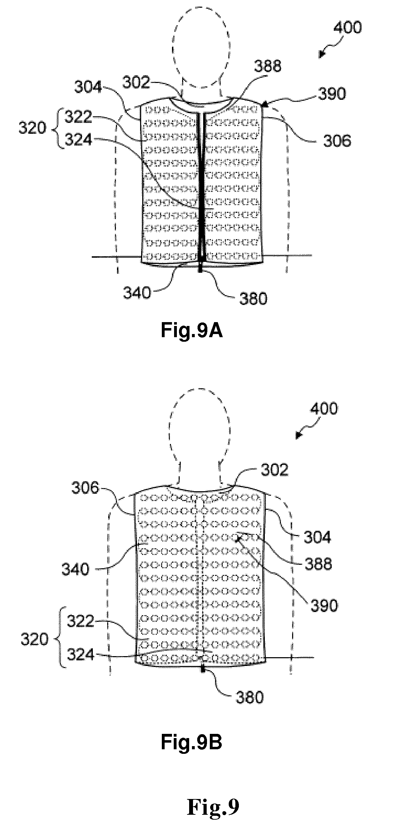

FIGs. 9A and 9B are a view illustrating a structure of an upper clothing 400 according to a modification of the Embodiment 3. FIG. 9A and FIG. 9B are a front view and a back view of the upper clothing 400.

As illustrated in FIGs. 9A and 9B, a plurality of LEDs may be disposed in place of the plurality of first light guides 368, the plurality of second light guides 372, and the plurality of third light guides 376, which are a plurality of optical fibers. In this case, the plurality of LEDs (light guides 388) are disposed so that light transmitting portions 390 (transmitting surfaces) are positioned across the inside surface of the upper clothing 300. Then, light through first light transmitting portions 382, second light transmitting portions 384, and third light transmitting portions 386 is transmitted to the entire chest of the wearer. The light transmitting portions 390 (transmitting surfaces) of the plurality of LEDs (light guides 388) may be configured to transmit light through the distal end only in an upper portion of the inside of the upper clothing 300 (a portion adjacent to a location that corresponds to a location of the wearer's pulmonary artery).

Other examples of the device for treating carbon monoxide poisoning include trocars that include a light emitter at the distal end, although the trocars are not shown herein.

Although in the embodiment, the upper clothing 300 that includes the light emitters 360 in the front body 320 and the back body 340 is described, the light emitters 360 may be disposed only in the front body 320, or the light emitters 360 may be disposed only in the back body 340.

The upper clothings 300 and 400 may be configured in a manner similar to a down jacket. In this case, the light transmitting portions come in intimate contact with the wearer, which allows efficient transmission of light.

As illustrated in the following experiments, the inventors of the present invention have developed the catheter 100 and the upper clothing 300 that can effectively dissociate carbon monoxide from CO-Hb and that can be used to remove carbon monoxide from the body. The catheter 100 and/or the upper clothing 300 of the present invention is expected to be used in combination with, for example, breathing in concentrated oxygen, hyperbaric oxygen therapy, and jet ventilation for treatment of carbon monoxide poisoning.

[Experiment 1]

In an Experiment 1, the effect of light exposure on the binding of carbon monoxide to hemoglobin vesicles was examined.

1. Preparation of Carbon Monoxide-Hemoglobin Vesicles (CO-HbV)

Carbon monoxide-hemoglobin vesicles (CO-HbV) were prepared in the following manner. First, HbV was prepared by enclosing hemoglobin purified from outdated human packed red blood cells with a phospholipid bilayer membrane. In particular, HbV was prepared by passing liquid prepared by adding mixed-lipid-particles and hemoglobin to saline through a membrane filter having a predetermined pore size under pressure (extrusion method). The prepared HbV had a particle diameter in the range of from 262 to 269 nm, a Hb concentration in the range of from 10.0 to 10.6 g/mL, a lipid concentration of from 6.9 to 7.2 g/mL, and an oxygen saturation of Hb of from 23 to 35 Torr. Then, CO-HbV was prepared by bubbling carbon monoxide through the HbV at 15 mL/min for 60 minutes.

2. Effect of Light Exposure on Binding of Carbon Monoxide to Hemoglobin Vesicles

First, it was examined whether carbon monoxide was dissociated from CO-HbV. Ten male Sprague Dawley (SD) rats that were 7 week old (with a body weight of from 255 to 282 g) were prepared. In the respective rats, 90% of the circulating blood was replaced with SALINHES (HES, 6% hydroxyethylated starch, Kyorin Pharmaceutical Co., Ltd.). And the subcutaneous tissue of the anterior chest of the respective rats was exposed. The 10 rats were randomly divided into a light exposure group of 5 rats and a light non-exposure group of 5 rats. Then, the CO-HbV obtained in the above manner was administered to the rats by intravenous injection at 25ml/kg. After administration of the CO-HbV, the anterior chest of the rats in the light exposure group was exposed to light having a wavelength in the range of from 400 to 1000 nm using FLG-2 light source device (illuminance of 27,000 lux at a measurement length of 100 mm and 12,000 lux at 150 mm, and luminance of 21,500,000 cd/m$^2$, Kyowa Optical Co., Ltd.). After 0 minute, 30 minutes, 60 minutes, and 90 minutes of the light exposure, arterial blood was collected from the respective rats. Then, the collected arterial blood was used to determine the saturation of the CO-HbV from the absorbance at the respective times.

3. Measurement of Carbon Monoxide Saturation in Blood

(1) Principle of Measurement of Carbon Monoxide Saturation

Carbon monoxide-hemoglobin (CO-Hb) and oxyhemoglobin (O$_2$-Hb) are known to have two absorption maximums. O$_2$-Hb is reduced by addition of hydrosulfite to provide reduced hemoglobin (Hb) having a single absorption maximum. In contrast, CO-Hb is not reduced by addition of hydrosulfite. Thus, the sample after addition of hydrosulfite has a composite absorption-spectrum derived from CO-Hb and Hb. As CO-Hb in blood increases, the single absorption maximum shifts to a shorter wavelength, and the absorption maximums derived from CO-Hb shifts to a longer wavelength. Thus, carbon monoxide saturation can be determined from the relationship between absorbance ratio and CO-Hb.

(2) Method for Calculating Carbon Monoxide Saturation

The carbon monoxide saturation was calculated from absorbance, as a ratio of the amount of CO-Hb after a predetermined amount of time has elapsed to the amount of CO-Hb (100%) immediately after light exposure. The carbon monoxide saturation was measured in the following manner. 10 mL of 0.1% aqueous sodium carbonate was added to 50 $\mu$L of blood to be tested and was allowed to stand for 15 minutes to prepare a sample to be tested. Then, the absorption spectrum of the sample to be tested was measured at a wavelength of from 500 to 600 nm. The sample to be tested had an absorption maximum of CO-Hb at 538 nm. Then, sodium hydrosulfite was added to the sample to be tested, and the absorption spectrum was measured again. The Hb had an absorption maximum at 555 nm. Then, the absorbance at 538 nm, which was an absorption maximum of the CO-Hb, and the absorbance at 555 nm, which was the absorption maximum of the Hb, of the sample to be tested were measured. Finally, $E_{538}/E_{555}$ was represented as $A_x$.

Oxygen was bubbled through the blood to be tested (for example, at 0.5 mL/min for 30 minutes) to prepare blood that contained oxygen at a saturated concentration and that was free of CO-Hb. The oxygen bubbling rate varies with the amount of the blood to be tested. Sodium hydrosulfite was added to diluted blood 1 that was prepared by adding 10mL of 0.1% aqueous sodium carbonate to 50 $\mu$L of the blood and was allowed to stand for 15 minutes to prepare a reference sample 1. The absorbance at 538 nm, which is an absorption maximum of CO-Hb, and the absorbance at 555 nm, which is the absorption maximum of Hb, of the reference sample 1 were measured. Then, $E_{538}/E_{555}$ was represented as $A_0$, which was 0.784.

Sodium hydrosulfite was added to diluted blood 2 that was prepared by adding 10 mL of 0.1% aqueous sodium carbonate to 50 $\mu$L of blood immediately after light exposure (after 0 minute of light exposure) and was allowed to stand for 15 minutes to prepare a reference sample 2. The absorbance at 538 nm, which is an absorption maximum of CO-Hb, and at 555 nm, which is the absorption maximum of Hb, of the reference sample 2 were measured. Then, $E_{538}/E_{555}$ was represented as $A_{100}$, which was 1.17.

Then, the carbon monoxide saturation (%) was calculated by the following

$$\text{formula:} \ (A_x - A_0)/\ (A_{100} - A_0) \times 100.$$

4. Results

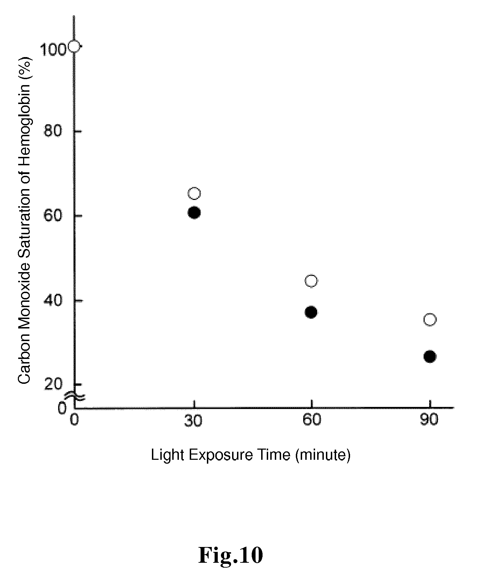

FIG. 10 is a graph illustrating the relationship between light exposure time and blood carbon monoxide saturation. In FIG. 10, the light exposure time (minutes) is taken along the abscissa. And carbon monoxide saturation (%) of hemoglobin is taken along the ordinate. The carbon monoxide saturation at the respective light exposure times is the average of five rats. In FIG. 10, white circle symbols represent the carbon monoxide saturation of the rats in light non-exposure group, while black circle symbols represent the carbon monoxide saturation of rats in light exposure group (at all wavelengths and 21,500,000 cd/m$^2$).

As illustrated in FIG. 10, the rats that were exposed to light at all wavelengths exhibited a lower blood carbon monoxide saturation, compared with the rats that were not exposed to light. This indicates that exposure of rat blood containing CO-Hb to light at all wavelengths results in dissociation of more carbon monoxide from hemoglobin.

[Experiment 2]

In an Experiment 2, the effect of the intensity of irradiated light on the binding of carbon monoxide to human hemoglobin in human blood was examined.

1. Effect of Light Exposure on Binding of Carbon Monoxide to Human Hemoglobin

First, blood was collected from adult male humans suffering from carbon monoxide poisoning. Part of the collected blood was exposed to light at an illuminance of 100,000 lux, 200,000 lux, and 500,000 lux. After 0 minute, 2 minutes, 4 minutes, 6 minutes, 8 minutes, 10 minutes, 12 minutes, 14 minutes, 16 minutes, 18 minutes, and 20 minutes of the exposure, part of the respective blood was collected and was examined for carbon monoxide saturation in the same manner as in the Experiment 1. The human CO-Hb has absorption maximums of 538 nm and 568 nm, and the human $O_2$-Hb has absorption maximums of 540 nm and 576 nm. The human Hb has an absorption maximum of 555 nm. $A_0$ and $A_{100}$ calculated from these values were 0.784 and 1.171, respectively.

2. Results

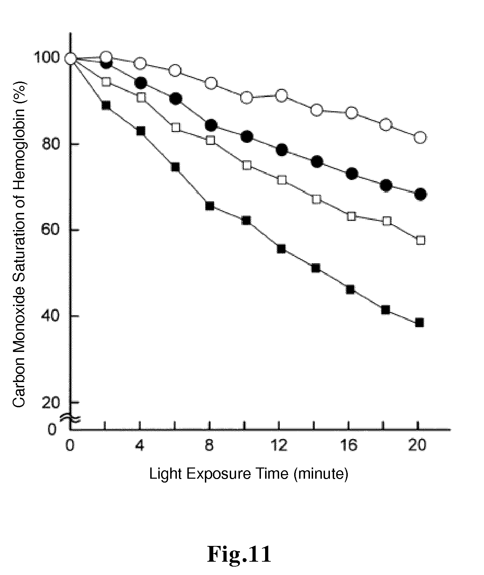

FIG. 11 is a graph illustrating the relationship between light exposure time and blood carbon monoxide saturation. In FIG. 11, the light exposure time (minutes) is taken along the abscissa. And carbon monoxide saturation (%) of hemoglobin is taken along the ordinate. In FIG. 11, white circle symbols represent carbon monoxide saturation in the case of no light exposure, black circle symbols represent carbon monoxide saturation in the case of exposure to light at 100,000 lux, white square symbols represent carbon monoxide saturation in the case of exposure to light at 200,000 lux, and black square symbols represent carbon monoxide saturation in the case of exposure to light at 500,000 lux.

As illustrated in FIG. 11, exposure of CO-Hb to a higher intensity of light leads to a lower carbon monoxide saturation of human blood, and exposure of CO-Hb to light for a longer period of time leads to a lower carbon monoxide saturation of human blood. This indicates that ease of dissociation of carbon monoxide of CO-Hb depends on the intensity of irradiated light and light exposure time.

[Experiment 3]

In an Experiment 3, the effect of the wavelength of irradiated light on the binding of carbon monoxide to human hemoglobin in human blood was examined.

1. Effect of Wavelength of Irradiated Light on Binding of Carbon Monoxide to Human Hemoglobin

Blood was collected from adult male humans suffering from carbon monoxide poisoning in the same manner as in the Experiment 1. Part of the collected blood is exposed to light at a wavelength of 680 nm using a light emitting diode. After 0 minute, 4 minutes, 8 minutes, 12 minutes, 16 minutes, and 20 minutes of the light exposure, part of the blood was collected, and the carbon monoxide saturation (%) was determined from the absorbance.

2. Results

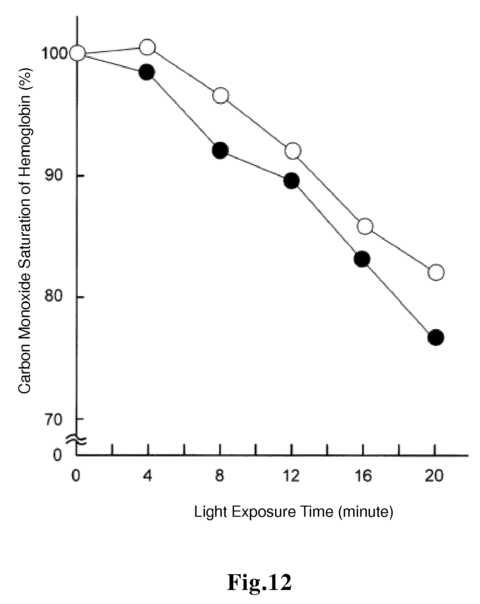

FIG. 12 is a graph illustrating the relationship between light exposure time and blood carbon monoxide saturation. In FIG. 12, the light exposure time (minutes) is taken along the abscissa. And carbon monoxide saturation (%) of hemoglobin is taken along the ordinate. In FIG. 12, white circle symbols represent carbon monoxide saturation in the case of exposure to light at all wavelengths, and black circle symbols represent carbon monoxide saturation in the case of exposure to light at a wavelength of 680 nm.

FIG. 12 indicates that exposure to light at a wavelength of 680 nm results in more effective dissociation of carbon monoxide from CO-Hb, compared with the case of exposure to light at all wavelengths. It has been also found that exposure to light having a wavelength in the range of from 600 to 750 nm can also result in effective dissociation of carbon monoxide from CO-Hb, although the results are not shown.

[Experiment 4]

In an Experiment 4, the effect of light exposure on the binding of carbon monoxide to human hemoglobin in porcine blood was examined in vitro.

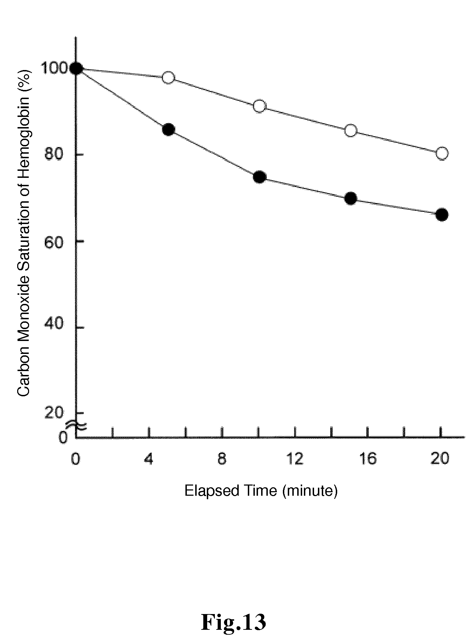

Carbon monoxide was bubbling through porcine blood (by placing 50 mL of porcine blood into a bag filled with 4.5 L of pure carbon monoxide gas and stirring the mixture well) to prepare blood containing carbon monoxide at a saturated concentration. The carbon monoxide bubbling ratio varies with the amount of the porcine blood. Oxygen was bubbling through part of the prepared blood (at 40 mL/min) while exposing the blood to a light at an illuminance of 600,000 lux. Oxygen was bubbling through another part of the prepared blood (at 40 mL/min) without light exposure. After 0 minute, 5 minutes, 10 minutes, 15 minutes, and 20 minutes of the light exposure, part of the respective blood was collected, and the carbon monoxide saturation was examined in the same manner as in the Experiment 1. The results are shown in FIG. 13. $A_0$ was 0.785, and $A_{100}$ was 1.155.

FIG. 13 is a graph showing the change in blood carbon monoxide saturation after initiation of the oxygen bubbling. In FIG. 13, elapsed time (minutes) is taken along the abscissa. And carbon monoxide saturation (%) of hemoglobin is taken along the ordinate. In FIG. 13, white circle symbols represent carbon monoxide saturation in the light non-exposure group, and black circle symbols represent the change in carbon monoxide saturation over time in the light exposure group (at all wavelengths and an illuminance of 600,000 lux).

As illustrated in FIG. 13, carbon monoxide saturation in porcine blood was lower in the case of light exposure (the light exposure group), compared with the case of no light exposure (the light non-exposure group). It has been also found that longer light exposure time leads to dissociation of more carbon monoxide.

[Experiment 5]

In an Experiment 5, the effect of light exposure on the binding of carbon monoxide to human hemoglobin in dog blood was examined in vitro.

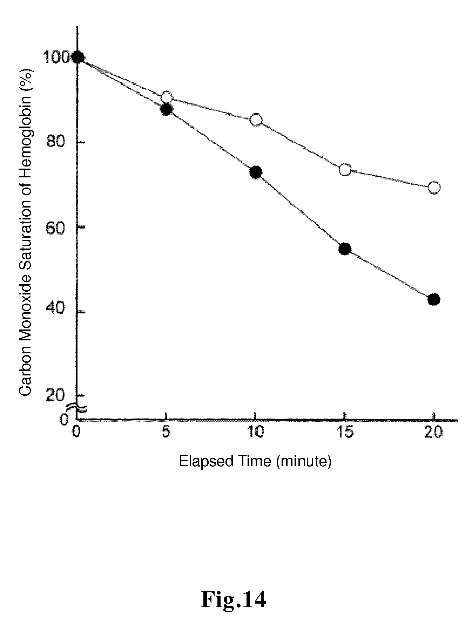

Carbon monoxide was added to dog blood (by placing 50 mL of dog blood into a bag filled with 4.5 L of pure carbon monoxide gas and stirring the mixture well) to prepare blood containing carbon monoxide at a saturated concentration. Oxygen was bubbling through part of the prepared blood (at 40 mL/min) while exposing the blood to light at an illuminance of 600,000 lux. Oxygen was bubbling through another part of the prepared blood (at 40 mL/min) without light exposure. After 0 minute, 5 minutes, 10 minutes, 15 minutes, and 20 minutes of the light exposure, part of the respective blood was collected, and the carbon monoxide saturation was examined in the same manner as in the Experiment 1. The results are shown in FIG. 14. $A_0$ was 0.813, and $A_{100}$ was 1.143.

FIG. 14 is a graph showing the change in carbon monoxide saturation in dog blood after initiation of the oxygen bubbling. In FIG. 14, elapsed time (minutes) is taken along the abscissa. And carbon monoxide saturation (%) of hemoglobin is taken along the ordinate. In FIG. 14, white circle symbols represent carbon monoxide saturation in the light non-exposure group, and black circle symbols represent the change in carbon monoxide saturation over time in the light exposure group (at all wavelengths and an illuminance of 600,000 lux).

As illustrated in FIG. 14, carbon monoxide saturation in dog blood was lower in the case of light exposure (the light exposure group), compared with the case of no light exposure (the light non-exposure group). It has been also found that longer light exposure time leads to dissociation of more carbon monoxide.

As described above, it was observed that light exposure resulted in dissociation of carbon monoxide from CO-Hb even in vitro experiments using human blood, dog blood, and porcine blood. Thus, it is expected that exposure of CO-Hb in vivo to light at a predetermined wavelength also results in dissociation of carbon monoxide from CO-Hb. It is also expected that use of a catheter and/or an upper clothing according to the present invention can leads to effective dissociation of carbon monoxide from CO-Hb. It is also expected that combination of the catheter and/or the upper clothing with hyperbaric oxygen therapy or breathing in concentrated oxygen provides further improved therapeutic effect.

The present application claims priority to Japanese Patent Application No. 2013-235804 filed on November 14, 2013. The entire contents of the application and the drawings therein are incorporated herein.

Industrial Applicability

For example, the catheter and the upper clothing of the present invention is useful as a catheter and an upper clothing as a device for treating carbon monoxide poisoning used to provide initial therapy for a patient suffering from acute carbon monoxide poisoning.

Description of the Reference Numeral

| | |
|---|---|
| 100, 200 | catheter |
| 120, 220 | catheter body |
| 121 | distal end portion |
| 122, 222 | first lumen |
| 123, 223 | second lumen |
| 124, 224 | third lumen |
| 126 | through-hole |
| 127 | cable |
| 130 | connector |
| 131 | light guide lumen |
| 132 | balloon lumen |
| 133 | pressure sensor lumen |
| 134 | light source connector |
| 135 | balloon inflation valve |
| 136 | syringe |
| 137 | connector |
| 140 | light emitter |
| 141 | light guide |
| 142 | light source |
| 143 | light transmitting portion |
| 160 | balloon |
| 180 | pressure sensor |
| 300, 400 | upper clothing |
| 302 | first opening |
| 304 | second opening |
| 306 | third opening |
| 320 | front body |
| 322 | first front body |
| 324 | second front body |
| 340 | back body |
| 360 | light emitter |

| 362 | first light emitter |
| 364 | second light emitter |
| 366 | third light emitter |
| 368 | first light guide |
| 370 | first light source |
| 372 | second light guide |
| 374 | second light source |
| 376 | third light guide |
| 378 | third light source |
| 380 | zipper |
| 382 | first light transmitting portion |
| 384 | second light transmitting portion |
| 386 | third light transmitting portion |
| 388 | light guide |
| 390 | light transmitting portion |

**Claims**

1. A device for treating carbon monoxide poisoning, the device comprising a light emitter that emits light having a wavelength in the range of from 600 to 750 nm.

2. An upper clothing for subjecting a patient suffering from carbon monoxide poisoning to light radiation to treat the carbon monoxide poisoning,
the upper clothing comprising the device for treating carbon monoxide poisoning according to claim 1,
wherein the light emitter emits light having a wavelength in the range of from 600 to 750 nm on the inside of a front body and/or on the inside of a back body.

3. The upper clothing according to claim 2,
wherein the light emitter comprises a plurality of light guides that allow light emitted by a light source disposed outside of the upper clothing to enter from one end and allow the entering light to exit from light transmitting portions at the other end, and
wherein the plurality of light transmitting portions are disposed on the inside of the front body and/or on the inside of the back body.

4. A catheter for insertion into a blood vessel of a patient suffering from carbon monoxide poisoning and for transmission of light into the blood,
the catheter comprising the device for treating carbon monoxide poisoning according to claim 1,
wherein the light emitter emits light having a wavelength in the range of from 600 to 750 nm at the distal end of the catheter.

5. The catheter according to claim 4, wherein the catheter comprises
a catheter body that has a distal end portion formed of a light-transmissive material and that comprises a first lumen, a second lumen, and a third lumen,
the light emitter that emits light having a wavelength in the range of from 600 to 750 nm to allow the light to exit through the first lumen from the distal end portion of the catheter body,
a balloon that is disposed around a circumferential surface of the distal end portion, that is in communication with the second lumen, and that guides the distal end portion to downstream blood flow in an inflated state, and
a pressure sensor that is disposed at the distal end of the distal end portion, that is connected to a cable inserted through the third lumen, and that measures intracardiac or intravascular pressure.

6. The catheter according to claim 5, wherein the distal end portion is a portion from the distal end of the catheter body to a position at 10 to 15 cm from the distal end.

7. The catheter according to claim 5 or claim 6, wherein the light emitted by the light emitter is transmitted outside of the catheter body in a distal-end side, a central portion, and a proximal-end side of the distal end portion, excluding the portion with the balloon disposed thereon.

8. The catheter according to any one of claims 5 to 7, wherein the light emitter comprises a plurality of light guides that allow light emitted by a light source disposed outside of the catheter body to enter from one end and allow the entering light to exit from light transmitting portions at the other end, and
wherein the plurality of light transmitting portions are disposed in the distal-end side, the central portion, and the proximal-end side of the distal end portion.

9. The catheter according to any one of claims 5 to 7, wherein the light emitter comprises a plurality of LEDs, and
wherein the plurality of LEDs are disposed in the distal-end side, the central portion, and the proximal-end side of the distal end portion.

10. The catheter according to any one of claims 4 to 9, wherein the light emitted by the light emitter has a light intensity of 1 mW or more.

**Fig.1**

Fig.2A

Fig.2B

Fig.2C

**Fig.2**

**Fig.3A**

**Fig.3B**

**Fig.3C**

**Fig.3**

Fig.4A

Fig.4B

Fig.4C

Fig.4

Fig.5A

Fig.5B

Fig.5C

**Fig.5**

**Fig.6A**

**Fig.6B**

**Fig.6C**

**Fig.6**

Fig.7A

Fig.7B

Fig.7C

Fig.7

**Fig.8A**

**Fig.8B**

**Fig.8**

**Fig.9A**

**Fig.9B**

**Fig.9**

**Fig.10**

**Fig.11**

**Fig.12**

**Fig.13**

**Fig.14**

## INTERNATIONAL SEARCH REPORT

| International application No. |
|---|
| PCT/JP2014/005744 |

A. CLASSIFICATION OF SUBJECT MATTER
*A61N5/06*(2006.01)i, *A61B17/00*(2006.01)i, *A61M25/10*(2013.01)i

According to International Patent Classification (IPC) or to both national classification and IPC

B. FIELDS SEARCHED

Minimum documentation searched (classification system followed by classification symbols)
A61N5/06, A61B17/00, A61M25/10

Documentation searched other than minimum documentation to the extent that such documents are included in the fields searched
Jitsuyo Shinan Koho          1922–1996   Jitsuyo Shinan Toroku Koho   1996–2015
Kokai Jitsuyo Shinan Koho    1971–2015   Toroku Jitsuyo Shinan Koho   1994–2015

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y<br>A | US 2013/0101464 A1 (SMYCZYNSKI, Mark S.),<br>25 April 2013 (25.04.2013),<br>paragraphs [0003], [0022], [0034]; fig. 1<br>(Family: none) | 1<br>2-10 |
| Y<br>A | JP 7-179357 A (Research Institute for<br>Production Development),<br>18 July 1995 (18.07.1995),<br>paragraphs [0009], [0015]<br>(Family: none) | 1<br>2-10 |
| A | JP 62-161382 A (Kei MORI),<br>17 July 1987 (17.07.1987),<br>page 2, upper right column, lines 15 to 20;<br>page 3, lower right column, lines 8 to 10; fig.<br>2 to 4<br>& US 4761047 A | 3-4 |

| ☒ Further documents are listed in the continuation of Box C. | ☐ See patent family annex. |
|---|---|

\* Special categories of cited documents:
"A"  document defining the general state of the art which is not considered    to be of particular relevance
"E"  earlier application or patent but published on or after the international filing date
"L"  document which may throw doubts on priority claim(s) or which is cited to establish the publication date of another citation or other special reason (as specified)
"O"  document referring to an oral disclosure, use, exhibition or other means
"P"  document published prior to the international filing date but later than the priority date claimed

"T"  later document published after the international filing date or priority date and not in conflict with the application but cited to understand the principle or theory underlying the invention
"X"  document of particular relevance; the claimed invention cannot be considered novel or cannot be considered to involve an inventive step when the document is taken alone
"Y"  document of particular relevance; the claimed invention cannot be considered to involve an inventive step when the document is combined with one or more other such documents, such combination being obvious to a person skilled in the art
"&"  document member of the same patent family

| Date of the actual completion of the international search<br>05 February 2015 (05.02.15) | Date of mailing of the international search report<br>24 February 2015 (24.02.15) |
|---|---|
| Name and mailing address of the ISA/<br>Japan Patent Office<br>3-4-3,Kasumigaseki,Chiyoda-ku,<br>Tokyo 100-8915,Japan | Authorized officer<br><br>Telephone No. |

Form PCT/ISA/210 (second sheet) (July 2009)

**INTERNATIONAL SEARCH REPORT**

International application No.

PCT/JP2014/005744

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2005-534409 A  (Miravant Medical Technologies, Inc.), 17 November 2005 (17.11.2005), paragraphs [0023], [0035], [0042]; fig. 7 & US 2004/0092830 A1     & WO 2004/012589 A2 | 4-8,10 |
| A | JP 2011-143258 A  (Unitika Ltd.), 28 July 2011 (28.07.2011), paragraphs [0025] to [0026] (Family: none) | 5 |
| A | JP 2008-526319 A  (Light Sciences Oncology, Inc.), 24 July 2008 (24.07.2008), paragraphs [0014], [0019]; fig. 16 & US 2007/0002582 A1     & WO 2006/074078 A1 | 9-10 |

Form PCT/ISA/210 (continuation of second sheet) (July 2009)

**EP 3 069 761 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2007045718 A

- JP 2013235804 A

### Non-patent literature cited in the description

- **E. NARIMATSU ; Y. ASAI.** 2. Carbon Monoxide Poisoning. *Clinic All-Round an extra issue,* 2002, vol. 51 (5), 748-751
- **K. ISEKI ; C. TASE.** Treatment of Acute Poisoning. *Japanese Journal of Intensive Care Medicine,* 2002, vol. 26 (5), 329-333
- **STEVEN L JACQUES.** Optical properties of biological tissues: a review. *Phys. Med. Biol.,* 2013, vol. 58, R37-R61

- **W. G. ZIJLSTRA ; A. BUURSMA ; O. W. VAN AS-SENDELFT.** *Visible and near infrared absorption spectra of human and animal Haemoglobin determination and application,* 2000, 58-59
- **W. G. ZIJISTRA ; A. BUURSMS ; W. P. MEEU-WSEN.** Absorption Spectra of Human Fetal and Adult Oxyhemoglobin, De-Oxyhemoglobin, Carboxyhemoglobin, and Methemoglobin. *CLINICAL CHEMISTRY,* 1991, vol. 37 (9), 1633-1638

31